Europäisches Patentamt

(19) European Patent Office

Office européen des brevets

(11) Numéro de publication: **0 188 942 B1**

## FASCICULE DE BREVET EUROPÉEN

(45) Date de publication du fascicule du brevet:
12.10.88

(51) Int. Cl.⁴: **A 61 F 13/18,** A 41 B 13/02, A 61 L 15/00

(21) Numéro de dépôt: **85402499.9**

(22) Date de dépôt: **16.12.85**

(54) **Matériau absorbant à base de fibres minérales.**

(30) Priorité: 17.12.84 FR 8419244

(43) Date de publication de la demande:
30.07.86 Bulletin 86/31

(45) Mention de la délivrance du brevet:
12.10.88 Bulletin 88/41

(84) Etats contractants désignés:
AT BE CH DE FR GB IT LI LU NL SE

(56) Documents cité:
EP-A-0 091 381
FR-A-1 550 233
US-A-2 477 403

(73) Titulaire: **ISOVER SAINT-GOBAIN, Les Miroirs 18, avenue d'Alsace, F-92400 Courbevoie (FR)**

(72) Inventeur: **Guyot, Daniel, 28, avenue Jean Jaurès, F-60290 Rantigny (FR)**
Inventeur: **Rieunier, Jean-Baptiste, 6, rue Gambetta, F-60100 Nogent Sur Oise (FR)**
Inventeur: **Conche, Michel, 5, allée des Glaieuls, F-60870 Villiers St-Paul (FR)**

(74) Mandataire: **Le Vaguerese, Sylvain Jacques, SAINT-GOBAIN RECHERCHE 39, quai Lucien Lefranc, F-93304 Aubervilliers Cedex (FR)**

0 188 942

## Description

L'invention est relative à un matériau fibreux minéral servant d'absorbant notamment pour les produits d'hygiène tels que les couches, les serviettes périodiques, les pansements et produits analogues.

Les matériaux destinés à ce type d'utilisation doivent satisfaire à des conditions multiples. Ils doivent notamment présenter une capacité d'absorption satisfaisante. Nous verrons comment en pratique cette grandeur est déterminée et les différentes considérations qui complètent cette notion.

Il est nécessaire également que ces matériaux soient d'une parfaite innocuité dans l'utilisation qui est envisagée, et de façon plus générale, réponde à toutes les exigences requises pour ce qui concerne les produits d'hygiène notamment ceux qui sont utilisés directement au contact du corps.

Le choix de ces matériaux résulte d'un ensemble de considérations dans lesquelles interviennent principalement leurs qualités propres pour ce type d'utilisation mais aussi leur coût relativement bas.

C'est cette dernière considération qui a conduit récemment au développent de l'utilisation des fibres de cellulose obtenues à partir du lois par des techniques papetières. Ces fibres sont préparées sous forme de ce qui est désigné sous le nom de "fluff", c'est-à-dire un enchevêtrement à faible masse volumique. Ce sont des fibres courtes irrégulières et qui, pour cette raison, forment un matériau présentant une cohésion faible et dans l'ensemble dont les qualités mécaniques sont modestes sinon mauvaises pour l'utilisation principalement envisagée. Manquant notamment de cohésion le fluff est normalement employé en combinaison avec d'autres matériaux. Ainsi le fluff servant d'absorbant pour les couches est toujours disposé à l'intérieur d'une enveloppe qui lui sert de support.

Par ailleurs, la capacité d'absorption de ces fluffs est limitée comme nous le verrons dans les exemples comparatifs fournis plus loin.

D'autres considérations font que le fluff ne donne pas entièrement satisfaction. Ainsi, en raison des fibres très courtes dont il est constitué, il résiste mal à la compression, que ce soit dans son conditionnement ou lors de son utilisation. Une fois comprimé, sa tendance naturelle à reprendre son volume initial est très faible et sa capacité d'absorption s'en trouve considérablement réduite.

De la même façon, l'affaissement sous charge à l'état humide qui constitue une caractéristique importante pour l'utilisation de ces matériaux est très importante, de l'ordre de 50 %.

Des dispositions ont été proposées pour remédier à cette insuffisance mécanique. Il s'agit notamment de renforcer le fluff en le mélangeant à d'autres matériaux. Outre qu'il n'est pas toujours commode d'obtenir une combinaison parfaitement homogène satisfaisante, la formation de ces matériaux composites fait perdre au fluff l'avantage que constitue son faible coût.

Par ailleurs, le fluff à l'état brut présente une teinte jaune ou brune qui n'est pas souhaitée pour les produits d'hygiène. Leur blanchiment par des techniques traditionnelles, notamment dans l'industrie papetière, est possible mais entraine des coûts supplémentaires d'autant plus élevés qu'il est nécessaire ensuite de procéder à une neutralisation poussée des résidus des agents chimiques utilisés.

Des produits organiques synthétiques ont également été proposés pour constituer ce type de matériau absorbant. Il s'agit notamment de produits à base de fibres cellulosiques greffées. Leur capacité d'absorption est habituellement très élevée mais leur cout de production les destine à des utilisations restreintes.

Il s'agit aussi des produits composites, constitués par un support fibreux dans lequel sont dispersés des particules ou poudres de produits de natures variées mais qui offrent une absorption particulièrement importante. En plus de leur cout élevé ces produits absorbants n'apportent aucune qualité mécanique à l'ensemble. En outre, à l'utilisation leurs propriétés absorbantes peuvent être limitées par leur tendance à former des agrégats.

Les feutres de fibres minérales ont déjà été proposés comme matériaux absorbants en particulier comme moyen pour lutter contre la pollution marine par les hydrocarbures. D'autres applications industrielles font appel également directement ou indirectement aux qualités absorbantes des feutres de fibres minérales, notamment aux feutres de fibres de verre.

Dans ces utilisations, les feutres choisis en raison de leur coût, sont du type de ceux qui ordinairement servent dans les domaines dans lesquels la production est la plus abondante, à savoir celui des feutres destinés à l'isolation. Les caractéristiques requises pour ces utilisations, combinées avec les impératifs de coût de production, font que ces produits ne sont pas utilisables en tant que produits d'hygiène corporelle.

En particulier, dans les produits commerciaux les plus répandus, les caractéristiques dimensionnelles des fibres et notamment la présence de fibres de diamètre de l'ordre de 10 micromètres ou plus, même en faible proportion, conduit au contact de la peau à des irritations qui les rendent impropres à cet usage.

Des fibres minérales longues, fines et bien régulières sont également connues et produites pour des applications très spécifiques. La production de ces fibres est ordinairement conduite par des techniques désignées sous le nom d'étirage à la flamme.

Suivant ces techniques, des filaments primaires solides d'un diamètre de l'ordre d'un millimètre sont introduits dans un courant gazeux à température et vitesse élevées. Sous l'action de ce courant, les filaments se ramollissent et s'étirent en fibres fines qui, dans certaines conditions, peuvent être pratiquement continues. Les feutres constitués à l'aide de ces fibres très fines présentent l'avantage d'offrir d'excellentes qualités isolantes pour des masses très réduites mais la technique de production est grande consommatrice d'énergie. Pour ces raisons, leurs principales utilisations sont limitées à des domaines spécialiés, par exemple l'industrie aéronautique, où les questions de poids constituent un élément d'appréciation primordial et où le coût

2

n'intervient que subsidiairement.

Quoiqu'il en soit, dans cette utilisation, l'aspect absorption de ces feutres n'apparait pas. Bien au contraire, l'on s'efforce de maintenir autant que possible les qualités hydrophobes par adjonction d'ensimages particuliers, par exemple à base de produits silicones.

Les inventeurs ont montré qu'il est possible d'utiliser des laines minérales pour constituer des produits absorbants et notamment des produits d'hygiène, dans des conditions de coûts comparables sinon plus avantageuses que celles correspondant à l'utilisation des matériaux traditionnels notamment du fluff cellulosique.

Les inventeurs ont aussi montré que dans ces utilisations, ces matériaux absorbants présentent encore d'autres avantages et notamment de meilleures propriétés mécaniques. Ils sont en outre normalement de couleur blanche.

La production de fibres fines en quantités abondantes par des moyens typiquement utilisés pour la production d'isolants, est à l'origine de l'invention selon laquelle à titre de matériau absorbant, et en particulier de matériau absorbant de liquides principalement aqueux, physiologiques, mais éventuellement aussi d'autres liquides, solvants, encres... on utilise un feutre de fibres minérales telles que des fibres de verre, dont la finesse est telle que ces fibres présentent une surface spécifique d'au moins 0,25 m2/g de fibres et de préférence plus de 0,5 m2/g.

De façon typique, ces surfaces spécifiques correspondent à des fibres dont le diamètre moyen est toujours inférieur à 5 micromètres et même inférieur à 2,5 micromètres. Mais les matériaux selon l'invention peuvent être encore sensiblement plus fins. Le diamètre moyen des fibres peut même être inférieur à 1 micromètre.

Autant que leur finesse, comme nous l'avons vu, il est imporant que ces fibres soient bien homogènes, et notamment que le matériau choisi soit exempt de particules infibrées ou recollées dont le diamètre pourrait atteindre ou dépasser 20 micromètres.

De façon générale, les matériaux absorbants selon l'invention sont constitués de fibres dont les diamètres sont compris entre 0,5 et 2 fois le diamètre moyen.

Il convient de souligner ici que les matériaux selon l'invention ont une structure fibreuse. Ou connait en effet des matériaux pulvérulents présentant une très grande surface spécifique et pour cette raison utilisés comme absorbants. Il va de soi que de tels matériaux ne peuvent conduire à une utilisation comparable à celles des matériaux selon l'invention. En dehors de la cohésion typique des matériaux fibreux, il est possible de distinguer les matériaux fibreux et les matériaux pulvérulents par des caractéristiques géométriques. Les matériaux fibreux présentent un allongement, c'est-à-dire un rapport longueur/diamètre qui est très élevé et dans tous les cas supérieur à 100 et généralement supérieur à 1000 alors que pour les matériaux pulvérulents ce rapport est voisin de l'unité et ne dépasse pas normalement 10.

En pratique comme nous le verrons à propos des exemples, la longueur des fibres utilisées est avantageusement de plusieurs centimètres. Compte tenu des finesses indiquées précédemment, les rapports d'allongement sont normalement de plusieurs milliers.

En dehors de leur finesse, les fibres doivent encore présenter certaines propriétés. En particulier, leur arrangement dans le feutre, arrangement qui dépend à la fois de leur longueur et aussi de la façon dont s'effectue la formation du feutre et les conditionnements ultérieurs du feutre ainsi formé, cet arrangement est avantageusement isotrope. En d'autres termes, les fibres à l'intérieur du matériau doivent être orientées de façon aléatoire; autrement dit encore, pour que le feutre offre les meilleures caractéristiques d'absorption, les fibres ne doivent se disposer suivant aucune direction dominante.

Dans le cas des produits absorbants, la distribution isotrope est favorable car elle correspond à des masses volumiques faibles et par suite à une capacité d'absorption, rapportée à la masse de fibres, élevée. En outre, cette même disposition favorise la reprise de l'épaisseur du feutre lorsque celui-ci a subi préalablement une compression. Cette propriété est avantageuse dans la mesure où le conditionnement de ces produits de faible masse volumique peut passer par une compression prolongée sans que les propriétés originales du produit soient affectées de façon substantielle. La capacité d'absorption dépend en effet de la porosité du feutre, c'est-à-dire de façon imagée, de l'espace "disponible" entre les fibres. Plus la masse volumique est faible, plus le produit est absorbant. Le fait que le produit reprenne bien son épaisseur après compression est donc une garantie de ses propriétés absorbantes et c'est à ce titre que cette particularité est importante selon l'invention.

Les qualités de "resistance" à la compression dont nous venons de parler interviennent aussi selon d'autres modalités. Pour les produits dont la capacité d'absorption est élevée, le problème se pose de l'affaissement du produit absorbant sous l'effet de la masse de liquide absorbé, ce qui tend à réduire les possibilités d'absorption. Si le produit résiste bien à la déformation, l'absorption est plus importante pour une même porosité initiale. Le phénomène est d'autant plus sensible que le produit s'étend sur une plus grande épaisseur.

Par ailleurs, dans l'utilisation même, les produits sont soumis à des compressions. Il est important que l'affaissement du matériau ayant absorbé le liquide, et soumis à cette charge ou à cette compression, soit aussi faible que possible. De cet affaissement dépend bien évidemment la capacité d'absorption effective.

Nous verrons à propos des essais des matériaux que la mesure normalisée de l'absorption sous charge se fait en imposant une compression de 2500 Pa. Dans ces conditions, les produits selon l'invention présentent avantageusement un affaissement à l'état humide qui n'est pas supérieur à 30 % et de préférence pas supérieur à 20 %.

On comprend que ces qualités soient favorisées non seulement par la distribution mais aussi par la longueur

3

des fibres, les fibres longues donnant plus facilement un effet de "ressort" au feutre.

Il va de soi que les propriétés qui viennent d'être indiquées ne sont atteintes que lorsque les fibres produites ne présentent pas dans leur structure de défauts susceptibles de les briser sous les efforts de la compression. En particulier, le diamètre des fibres ne doit pas varier dans des proportions importantes le long d'une même fibre. Néanmoins, si leur diamètre doit être sensiblement constant, ces fibres peuvent avantageusement former des boucles ou des ondulations, lesquelles favorisent l'enchevêtrement et l'isotropie. Cette dernière caractéristique constitue une différence des fibres minérales utilisées selon l'invention vis-à-vis de celles produites selon les techniques d'étirage à la flamme dont il a été question plus haut, ces dernières présentant des formes peu ondulées.

Les qualités isotropes des feutres peuvent être appréciées expérimentalement en mesurant par exemple la résistance qu'ils opposent au passage de l'air suivant une direction perpendiculaire à leurs faces et selon une direction parallèle à ces faces. Les faces en question sont détermnées à la réception des fibres produites. Une de ces faces est au contact du convoyeur de réception, l'autre correspond à la limite supérieure de la nappe de fibres déposée sur ce convoyeur. Le feutre est d'autant plus isotrope que ces deux résistances sont proches l'une de l'autre. Il est préférable d'utiliser des feutres dont le rapport de résistance au passage de l'air, ou rapport d'isotropie, n'est pas supérieur à 2.

S'il est avantageux pour l'absorption d'avoir des structures fibreuses isotropes, en pratique on constate néanmoins souvent que l'isotropie parfaite n'est pas atteinte. Les modes traditionnels de réception des fibres qui viennent d'être formées, à savoir "la filtration" des fibres sur un tapis convoyeur perméable aux gaz et retenant les fibres véhiculées par des courants gazeux, favorisent une disposition dans les plans parallèles au convoyeur de réception.

Cette tendance naturelle est contrariée par la dimension des fibres et surtout par leurs ondulations et boucles comme indiqué précédemment.

Il faut encore préciser qu'une orientation privilégiant la disposition dans des plans parallèles aux faces du feutre produit peut, à condition de n'être pas exclusive, présenter certains avantages. Ainsi on constate que la tendance à la "stratification" selon des plans parallèles au convoyeur conduit à des produits dans lesquels la diffusion du liquide absorbé est relativement plus rapide que dans un matériau parfaitement isotrope. Selon les qualités requises, c'est-à-dire suivant l'utilisation envisagée, il peut donc être avantageux de choisir un matériau partiellement stratifié pour améliorer la rapidité de diffusion même si ceci est obtenu en réduisant un peu de la capacité d'absorption.

En raison de la finesse et des qualités mécaniques des fibres constituant les matériaux selon l'invention, ceux-ci peuvent atteindre des volumes spécifiques relativement importants à l'état non comprimé. De façon typique, un matériau absorbant à base de fibres de verre selon l'invention présente un volume de plus de 15 $cm^3$ par gramme de fibre et de préférence supérieur à 20 $cm^3/g$, et peut atteindre et même dépasser 50 $cm^3/g$.

Cette autre caractéristique distingue aussi les matériaux selon l'invention, des matériaux absorbant pulvérulents dont il a été question plus haut, dont le volume spécifique est beaucoup plus faible.

Compte tenu de ce volume spécifique élevé, la capacité d'absorption de ces matériaux est ordinairement supérieure à 15 g d'eau par gramme de fibre et, avantageusement, supérieure à 20 g/g et même peut dépasser 25 g/g, lorsque cette capacité est mesurée sans charge. Lorsque la mesure est reproduite avec des échantillons soumis à une charge normalisée de 2500 Pa, la capacité d'absorption des matériaux selon l'invention est supérieure à 12 g d'eau par gramme de fibre et pour les produits préférés, supérieure à 15 g/g.

Dans les essais rapportés ci-après quatre matériaux selon l'invention, désignés respectivement sous les références A, B, C et D sont comparés à un fluff utilisé industriellement pour constituer des couches.

Le mode de production de la laine de verre est du type décrit dans la demande de brevet européen publiée sous le n° 0 091 381.

Dans ce mode de production, du verre fondu provenant d'un four de fusion s'écoule dans un dispositif de centrifugation.

Avantageusement, la composition de verre utilisée correspond à celle définie dans la demande de brevet européen précitée, à savoir en proportion pondérale:

| | | | | | | |
|---|---|---|---|---|---|---|
| $SiO_2$ | 61 | - | 66 | $Na_2O$ | 12,55 - 16,5 | |
| $Al_2O_3$ | 2,5 | - | 5 | $K_2O$ | 0 - 3 | |
| CaO | 6 | - | 9 | $B_2O_3$ | 0 - 7,5 | |
| MgO | 0 | - | 5 | $Fe_2O_3$ | moins de 0,6 | |

Le verre fondu est conduit à une température de l'ordre de 1500°C, sous forme d'une coulée continue, dans un organe distributeur disposé à l'intérieur de l'organe centrifugeur forment les fibres. Le verre divisé en gros filets (3 à 4 mm de diamètre) est projeté sur la paroi périphérique interne du centrifugeur.

Le centrifugeur présente un nombre élevé d'orifices par lesquels le verre s'échappe sous l'effet de la force centrifuge. Les orifices sont de faibles dimensions, de l'ordre de 1 mm de diamètre.

Les conditions d'alimentation, notamment température et débit du verre, température du centrifugeur, sont réglées de façon à maintenir un écoulement continu du matériau à partir des orifices de centrifugation.

Les fins filaments projetés à partir des orifices de la paroi du centrifugeur pénètrent dans un courant gazeux chaud à vitesse élevée, établi de façon à longer la paroi du centrifugeur suivant une direction voisine de l'axe de celui-ci.

Les filaments sont entraînés et étirés par ces gaz chauds provenant traditionnellement d'un brûleur à combustion interne.

Les fibres formées se figent au contact avec l'air ambiant. Elles sont récupérées sur un convoyeur perméable aux gaz. Sur ce convoyeur elles se déposent sous forme d'un feutre à faible masse volumique dont l'épaisseur est fonction du débit du dispositif de formation des fibres, de la largeur du convoyeur et de la vitesse de défilement de celui-ci. Eventuellement, les conditions peuvent être réglées de façon que le feutre recueilli puisse être utilisé directement pour former la nappe de matériau absorbant selon l'invention après découpe aux dimensions adéquates.

Les matériaux fibreux absorbants selon l'invention peuvent être utilisés tels quels. Il est possible aussi de traiter les fibres pour leur conférer des propriétés encore améliorées. Un traitement éventuel consiste par exemple à pulvériser sur le trajet des fibres, avant leur réception sur le convoyeur, une composition d'ensimage.

Contrairement aux feutres d'isolation, les nappes fibreuses formées conformément à l'invention ne sont habituellement pas liées par des résines. La cohésion naturelle de la nappe de fibres est normalement suffisante pour l'utilisation envisagée. Par ailleurs, l'adjonction d'une résine liante, en plus du coût supplémentaire qu'elle occasionne, se traduit habituellement par une diminution de l'hydrophilie des fibres. Pour toutes ces raisons, il est préférable de ne pas lier les fibres contrairement à ce qui est pratiqué dans le domaine des feutres d'isolation.

Le cas échéant néanmoins, une faible proportion d'un agent liant qui ne diminue pas l'hydrophilie, par exemple une composition à base d'amidon, peut être utilisée.

D'autres composés peuvent être également pulvérisés sur les fibres par exemple pour en modifier le toucher.

Les propriétés des nappes de matériau absorbant selon l'invention varient sensiblement, comme nous le verrons dans les résultats donnés plus loin selon les conditions de leur production. Si, de façon générale comme nous l'avons déjà indiqué, la technique mise en oeuvre est celle décrite dans la demande de brevet européen citée, des conditions spécifiques peuvent être préférées pour atteindre les meilleurs résultats.

Compte tenu des applications envisagées, on choisit en effet dans la mise en oeuvre de ces techniques les conditions qui favorisent notamment la finesse des fibres et, dans une mesure moindre, leur longueur même si ce choix conduit à une production relativement moins abondante.

De façon pratique, un des avantages de la technique décrite dans la demande précitée est de modérer l'action des courants gazeux dans le processus aboutissant à l'étirage des fibres. L'expérience montre en effet pour ce type de technique, que pour un débit déterminé et pour une finesse de fibre donnée, la longueur des fibres dépend des conditions de soufflage. Plus le courant gazeux est intense, plus les fibres sont fines mais également plus elles sont courtes. Le fait d'obtenir un bon étirage sans accroitre le soufflage, comme prévu suivant la technique en question, permet donc d'avoir des fibres fines, en quantité relativement abondante et avec des longueurs satisfaisantes pour l'usage considéré.

Comme il ressort des indications précédentes, des matériaux utilisables selon l'invention peuvent être préparés dans des conditions sortant du cadre défini dans la demande citée, mais ceci au détriment du rendement et par suite du coût de production qui reste un facteur important pour que ces matériaux soient avantageusement substitués aux matériaux absorbants traditionnels.

A titre indicatif, les matériaux préparés l'ont été en réglant le régime du brûleur générateur de gaz chauds de façon que l'émission soit faite à une vitesse d'environ 100 à 200 m/s et à une température de 1380 à 1550°C. La température du centrifugeur, dans ces conditions, s'établit entre 920 et 1050°C.

Il va de soi également que pour un même dispositif, c'est-à-dire notamment pour le même nombre d'orifices et par suite le même nombre de filaments, la masse de fibres produite varie en fonction de leur finesse. Plus les fibres sont fines, plus la masse est faible.

Le tableau suivant donne, pour les quatre matériaux selon l'invention, la masse de fibres produite par jour pour chaque dispositif de centrifugation. Le tableau comporte également la surface spécifique de chacun des matériaux obtenus et leur volume massique.

| fibres | débit en $10^3$ kg/j | surface spécifique $m^2/g$ | volume massique $cm^3/g$ | sous charge 2500 Pa |
|--------|--------|--------|--------|--------|
| A | 0 | 0,30 | 24 | 19 |
| B | 10 | 0,75 | 32 | 20,3 |
| C | 7 | 1,25 | 35 | 22,7 |
| D | 4 | 2,6 | 38,5 | 25,6 |

Les fibres produites sont d'une grande régularité et pratiquement dépourvues de particules hétérogènes telles que celles qui peuvent se trouver dans les produits d'isolation et qui proviennent de défauts de fibrage.

Les fibres récupérées sans traitement sont blanches et douces au toucher. L'impression est comparable à celle d'une ouate de coton. Les fibres dont la longueur moyenne est de l'ordre de 3 à 4 cm sont souples.

A titre de comparaison les fibres fluff de cellulose sont plus épaisses, beaucoup plus courtes et leur volume massique légèrement inférieur est de l'ordre de 20 $cm^3/g$.

A partir de ces différents matériaux, des éprouvettes sont préparées suivant des dimensions bien définies

# 0 188 942

pour tester les qualités absorbantes.

Dans une première série d'essais, des échantillons sont préparés dans les conditions proposées par la norme scandinave SCAN C 33-80 pour les mesures effectuées habituellement pour le fluff.

Le principe de la méthode est le suivant.

Les échantillons de matériau absorbant préalablement mis en condition (masse, forme, température, ...) sont disposés sur une grille.

La grille comprimée sous une charge uniformément répartie correspondant à 2500 Pa est placée au contact d'une nappe d'eau. L'échantillon s'imbibe. Lorsque l'équilibre est atteint, la grille est relevée. La quantité d'eau absorbée est déterminée par la différence de poids de l'échantillon avant et après contact avec l'eau.

Au cours de ces essais, on détermine simultanément le temps nécessaire pour que l'eau progresse depuis la face de contact jusqu'à la face supérieure du matériau.

Les résultats de ces essais sont reportés dans le tableau suivant.

| fibres | absorption g/g sous 2500 Pa | temps de mouillage (s) | vitesse de diffusion mm/s |
|---|---|---|---|
| fluff | 9 - 11,6 | 4,8 | 4,6 |
| A | 12 - 15 | 8 - 12 | 2,3 - 3,4 |
| B | 14 - 19 | 8 - 12 | 2,4 - 3,6 |
| C | 19 - 21 | 12 - 14 | 2,3 - 2,7 |
| D | 13 - 16 | 20 | - |

A partir des résultats précédents, on constate de façon générale que les matériaux selon l'invention sont très sensiblement meilleurs que le fluff pour ce qui concerne la capacité d'absorption.

La capacité d'absorption pour les meilleurs essais est pratiquement doublée et pour les fins bons est augmentée d'environ 50 %. Le progrès est donc considérable.

La comparaison des quatre produits selon l'invention met en évidence la relation existant entre la surface spécifique et la capacité d'absorption. Plus la surface est grande, plus élevée est la masse d'eau absorbée pour la même masse de fibres.

Cette tendance est cependant limitée par l'affaissement relatif de l'échantillon sous l'effet de la masse de liquide absorbée. Une mesure des hauteurs de l'échantillon à sec et imbibé montre bien ce phénomène. C'est la raison pour laquelle l'échantillon D, constitué des fibres les plus fines, présente une absorption moins forte que l'échantillon C. La différence d'absorption traduit exactement la différence de hauteur. Si l'échantillon D sec est plus volumineux que l'échantillon C, il subit un affaissement qui l'amène, à l'état humide, à un volume inférieur à celui de l'échantillon C. Même pour cet échantillon, il faut remarquer que la capacité d'absorption reste supérieure à celle du fluff.

Si dans ce tableau le temps de mouillage des échantillons selon l'invention parait plus important, cela ne veut pas dire que l'absorption se fait plus lentement. En effet, le temps de mouillage correspond au temps nécessaire pour que le liquide absorbé à la base de l'échantillon atteigne le point le plus haut. Etant donné que les échantillons selon l'invention sont en fin d'essai plus hauts que ceux du fluff, ou encore qu'ils absorbent davantage de liquide il n'est pas surprenant que ce temps de moiillage soit plus long. Si l'on rapporte ce temps à la hauteur de l'échantillon ou à la masse de liquide absorbée, on constate alors que la vitesse de diffusion (ou la vitesse d'absorption) est tout à fait du même ordre de grandeur que celle du fluff. La légère différence qui peut être constatée résulte du fait que la vitesse de diffusion dans l'échantillon n'est pas indépendante de la hauteur de l'échantillon. La diffusion est plus rapide au niveau du contact avec la surface du liquide et décroit au fur et à mesure que le point considéré est plus élevé par rapport à cette surface. Des mesures faites de la diffusion du liquide à des hauteurs identiques dans des échantillons de fluff d'une part et d'absorbant selon l'invention d'autre part, montrent une grande similitude pour le fluff présentant la plus grande vitesse d'absorption. En moyenne les mêmes mesures faites sur des fluffs de diverses origines font apparaître, dans l'ensemble, une vitesse de diffusion qui est sensiblement moindre qu'avec les produits absorbants selon l'invention.

Compte tenu des résultats qui viennent d'être présentés, il ressort clairement que les matériaux selon l'invention conviennent bien pour la confection de produits absorbants tels que les couches et produits analogues dont il a été question au début de ce texte.

Dans ce type d'utilisation, les matériaux absorbants en question peuvent être utilisés seuls ou en mélange avec d'autres produits absorbants. Il est notamment possible de procéder à un mélange de fibres de verre, telles que celles décrites précédemment, avec des fluffs cellulosiques utilisés traditionnellement.

Il est possible aussi, le cas échéant, de mélanger des fibres de verre de caractéristiques différentes pour conférer au mélange à la fois les meilleures qualités d'absorption liées à la finesse et une bonne résistance à l'affaissement à l'état humide.

Le produit B selon l'invention est mélangé dans un "fluffeur" à des fibres fluff dans diverses proportions. L'on suit les variations des propriétés du produit ainsi préparé en fonction de la proportion pondérale des fibres de verre.

| % fibres de verre | volume massique cm³/g | capacité d'absorption sous charge 2500 Pa |
|---|---|---|
| 0 | 24 | 8,0 |
| 10 | 26 | 9,2 |
| 20 | 28 | 10,0 |
| 30 | 29 | 10,2 |
| 50 | 30,5 | 12,2 |
| 100 | 33 | 15,8 |

Les résultats de ce tableau montrent une bonne concordance avec ceux correspondant aux essais précédents. Ils montrent surtout que les fibres de verre selon l'invention peuvent être utilisées avec d'autres matériaux absorbants pour en modifier les caractéristiques, en particulier la capacité d'absorption.

A côté de l'amélioration des propriétés d'absorption, la fibre de verre est surtout mélangée au fluff pour modifier ses propriétés mécaniques. Une des caractéristiques du fluff est, comme nous l'avons indiqué, son manque de cohésion qui provient de ce qu'il est constitué de fibres extrêmement courtes.

L'amélioration des qualités du fluff permet de modifier les conditions d'utilisation. Dans la pratique, en effet, pour la constitution de couches absorbantes par exemle, le fluff doit nécessairement être introduit dans des enveloppes qui confèrent à l'ensemble les caractéristiques dimensionnelles nécessaires et le choix du matériau constituant cette enveloppe est déterminé par cet imperatif de résistance. L'utilisation d'un matériau tel que celui de l'invention permet sinon de se dispenser de l'enveloppe, d'être moins limité dans le choix des caractéristiques de celle-ci.

Dans les conditions de production qui ont été indiquées précédemment, il est en outre important de souligner que le coût du matériau absorbant constitué de fibres de verre en fait un produit avantageusement substituable aux matériaux traditionnels, tel que le fluff. Même si par unité de poids le coût de production est un peu plus élevé, les performances atteintes en ce qui concerne l'absorption permettent d'utiliser une masse beaucoup plus réduite de fibres pour le même effet. Par ailleurs, comme nous l'avons vu, le choix des matériaux selon l'invention permet d'améliorer sensiblement d'autres propriétés, notamment les propriétés mécaniques.

A ce sujet, il faut souligner que les essais d'absorption rapportés ci-dessus sont conduits sur des échantillons dont les qualités n'ont pas été altérées par le conditionnement et le stockage. Dans la pratique, il va de soi qu'au cours de ces opérations le matériau absorbant est soumis à des contraintes, notamment il subit des compressions. "L'élasticité" des feutres constitués de fibres de verre permet à ces matériaux une bonne reprise des propriétés initiales lorsque ces contraintes disparaissent. En conséquence, les propriétés absorbantes sont peu ou pas modifiées. Au contraire, les matériaux absorbants à base de fluff ne reprennent pas leurs propriétés initiales.

On a vérifié que les propriétés absorbantes des produits selon l'invention ne sont pas détériorées lorsqu'ils sont soumis à une compression préalable relativement forte ayant pour but de simuler les modifications subies par exemple en cours d'entreposage.

Pour cela des échantillons du produit C sont soumis à une compression dans le sens de leur épaisseur pendant une heure. On mesure la réduction d'épaisseur correspondant à cette compression. La compression est relachée pendant 24 heures et l'on procède ensuite à une mesure de capacité d'absorption dans les conditions de la norme scandinave SCAN C 33-80 (c'est-à-dire sous compression de 2500 Pa).

On constate que, même pour les traitements de compression préalable les plus sévères, la capacité d'absorption des produits selon l'invention n'est que relativement peu altérée.

| compression préalable | épaisseur de l'échantillon (mm) | % réduction d'épaisseur | absorption sous 2500 Pa (g/g) |
|---|---|---|---|
| 2500 | 37 | 0 | 19 |
| 5000 | 27 | 27 | 19 |
| 10000 | 20 | 46 | 18,5 ( - 2,6 %) |
| 20000 | 14 | 62 | 17,5 ( - 8 %) |

L'avantage procuré par les produits absorbants selon l'invention ne réside pas uniquement dans leur capacité de rétention des liquides; on observe également une résistance mécanique accrue qui rend leur mise en oeuvre plus commode.

Cette résistance mécanique a été étudiée au moyen d'un test de pénétration effectué sur des échantillons préparés comme pour l'essai SCAN C 33-80. L'échantillon qui se présente sous forme d'un disque est maintenu à sa périphérie. Un piston progressant de 20 mm/min est appliqué au centre du disque et détermine la force nécessaire pour perforer l'échantillon. En reproduisant les mêmes conditions, on observe une résistance de 3 à 4 fois supérieure pour les produits selon l'invention par rapport aux fluffs traditionnels.

## Revendications

1. Matériau absorbant sous forme de nappe de fibres minérale enchevêtrées dont la surface spécifique est au moins 0,25 m²/g.

2. Matériau selon la revendication 1 dans lequel les fibres présentent une surface spécifique comprise entre 0,5 et 1,5 m²/g.

3. Matériau selon la revendication 1 ou la revendication 2 dans lequel le rapport de la longueur moyenne des fibres à leur diamètre moyen est supérieur à 100.

4. Matériau selon l'une des revendications 1 à 3 dans lequel le volume spécifique à l'état non comprimé est au moins égal à 15 cm³ par gramme de fibres.

5. Matériau selon la revendication 4 dans lequel le volume spécifique est supérieur à 20 cm³/g.

6. Matériau selon l'une des revendications précédentes dont la capacité d'absorption par capillarité, sous une pression de 2500 Pa, est supérieure à 12 g d'eau par gramme de fibres.

7. Matériau selon la revendication 6 dont la capacité d'absorption par capillarité, sous une pression de 2500 Pa, est supérieure à 15 g d'eau par gramme de fibres.

8. Matériau selon la revendication 7 dont l'affaissement sous une charge de 2500 Pa est inférieur à 20 %.

9. Procédé de fabrication d'un matériau selon l'une quelconque des revendications précédentes dont les fibres sont produites par passage du verre fondu par les orifices disposés à la périphérie d'un centrifugeur, les fibres produites véhiculées par un courant gazeux étant recueillies sous forme d'un feutre sur un organe de réception, le feutre recueilli étant éventuellement soumis à une opération de cardage pour accroître son volume massique.

10. Nappe absorbante comprenant un matériau selon l'une quelconque des revendications 1 à 8.

11. Nappe absorbante selon la revendication 10 dans laquelle le matériau constitué de fibres de verre est mélangé avec un autre matériau absorbant, dans une proportion pondérale qui n'est pas inférieure à 10 % / 90 %.

12. Nappe absorbante selon la revendication 11 dans laquelle les fibres de verre sont mélangées avec des fibres cellulosiques dans une proportion pondérale qui n'est pas inférieure à 50 % / 50 %.

## Patentansprüche

1. Absorbierendes Material in Form einer Bahn aus verfilzten Mineralfasern, deren spezifische Oberfläche wenigstens gleich 0,25 m²/g ist.

2. Material nach Anspruch 1, bei dem die Fasern eine spezifische Oberfläche zwischen 0,5 und 1,5 m²/g aufweisen.

3. Material nach Anspruch 1 oder Anspruch 2, bei dem das Verhältnis zwischen der mittleren Länge der Fasern zu deren mittlerem Durchmesser größer ist als 100.

4. Material nach einem der Ansprüche 1 bis 3, bei dem die reziproke Dichte im nichtkomprimierten Zustand wenigstens 15 cm³/g Fasern beträgt.

5. Material nach Anspruch 4, bei dem die reziproke Dichte größer ist als 20 cm³/g.

6. Material nach einem der vorhergehenden Ansprüche dessen Absorbtionsfähigkeit durch Kapillarwirkung unter einem Druck von 2500 Pa größer ist als 12 g Wasser pro Gramm Fasern.

7. Material nach Anspruch 6, dessen Absorbtionsfähigkeit durch Kapillarwirkung unter einem Druck von 2500 Pa größer ist als 15 g Wasser pro Gramm Fasern.

8. Material nach Anspruch 7, dessen Zusammendrückbarkeit unter einer Last von 2500 Pa geringer ist als 20 %.

9. Verfahren zur Herstellung eines Materials nach einem der vorhergehenden Ansprüche, dessen Fasern durch Hindurchtreten von Glasschmelze durch am Umfang einer Zentrifuge angeordnete Öffnungen hergestellt werden, wobei die von einem Gasstrom beförderten hergestellten Fasern in Form eines Filzes auf einem Aufnahmeorgan gesammelt werden, und der aufgefangene Filz gegebenenfalls einem Kardierungsvorgang unterworfen wird, um seine Rohdichte (volume massique) zu erhöhen.

10. Absorbierende Bahn mit einem Material nach wenigstens einem der Ansprüche 1 bis 8.

11. Absorbierende Bahn nach Anspruch 10, in der das aus Glasfasern gebildete Material mit einem anderen absorbierenden Material vermischt ist, in einem Verhältnis der Anteile, das nicht kleiner ist als 10 Gew.-% / 90 Gew.-%.

12. Absorbierende Bahn nach Anspruch 11, in der die Glasfasern mit zelluloseartigen Fasern in einem Verhältnis vermischt sind, das nicht geringer ist als 50 Gew.-% / 50 Gew.-%.

## Claims

1. Absorbent material in the form of a sheet of tangled mineral fibres of which the specific surface area is at least 0.25 m²/g.

2. Material according to claim 1 in which the fibres have a specific surface area between 0.5 and 1.5 m²/g.

3. Material according to claim 1 or claim 2 in which the ratio of the average length of the fibres to their average diameter is greater than 100.

4. Material according to any one of claims 1 to 3 in which the specific volume in the non-compressed state is at least equal to 15 cm³ per gram of fibres.

5. Material according to claim 4 in which the specific volume is greater than 20 cm³/g.

6. Material according to any one of the preceding claims in which the capillary absorbtion capacity, under a pressure of 2500 Pa, is greater than 12 g of water per gram of fibres.

7. Material according to claim 6 of which the capillary absorbtion capacity, under a pressure of 2500 Pa, is greater than 15 g of water per gram of fibres.

8. Material according to claim 7 in which sagging under a force of 2500 Pa is less than 20 %.

9. Process for the manufacture of material according to any one of the preceding claims in which the fibres are produced by passing molten glass through orifices disposed at the periphery of a centrifuge, the resulting fibres conveyed by a gaseous current being collected in the form of a felt on a receiver, and the collected felt being optionally fitted to a carding operation in order to increase volume per unit mass.

10. Absorbent sheet comprising a material according to any one of claims 1 to 8.

11. Absorbent sheet according to claim 10 in which the material formed from glass fibres is mixed with another absorbent material, in a proportion by weight which is not less than 10% / 90%.

12. Absorbent sheet according to claim 11 in which the glass fibres are mixed with cellulosic fibres in a proportion by weight which is not less than 50% / 50%.